# EUROPÄISCHE PATENTSCHRIFT

(11) **EP 2 878 303 B1**
(45) Veröffentlichungstag und Bekanntmachung des Hinweises auf die Patenterteilung: **15.11.2017**
(21) Anmeldenummer: 13005575.9
(22) Anmeldetag: 29.11.2013
(51) Int. Cl.: A61K 36/34, A61K 36/15, A61K 36/28, A61Q 13/00, A61K 36/85, A61K 36/87, A61K 36/185, A61K 36/38, A61K 36/54, A61K 36/63, A61K 8/34, A61K 8/92, A61K 9/00, A61P 39/00

(54) **Zusammensetzung für die Aromatherapie, Aromaheilkunde und Aromatologie**
Compound for aromatherapy, aromatherapy medicine and aromatology
Composition pour l'aromathérapie, la médecine aromatique et l'aromatologie

(43) Veröffentlichungstag der Anmeldung: 03.06.2015
(73) Patentinhaber: Hefti, Doris, 6343 Rotkreuz (CH)
(72) Erfinder: Hefti, Doris, 6343 Rotkreuz (CH)
(74) Vertreter: Schaad, Balass, Menzl & Partner AG

(56) Entgegenhaltungen:
- FR-A1- 2 979 827
- STEPHANIE DE RAPPER ET AL: "The In Vitro Antimicrobial Activity of Lavandula angustifolia Essential Oil in Combination with Other Aroma-Therapeutic Oils", EVIDENCE-BASED COMPLEMENTARY AND ALTERNATIVE MEDICINE : ECAM, Bd. 17, Nr. 5, 1. Januar 2013 (2013-01-01) , Seiten 399-10, XP55106509, United States ISSN: 1741-427X, DOI: 10.1111/j.1750-3841.2008.00790.x
- DATABASE WPI Week 200709 Thomson Scientific, London, GB; AN 2007-087378 XP002721406, & KR 2006 0029948 A (ENPRANI CO LTD) 7. April 2006 (2006-04-07)
- DATABASE WPI Week 200306 Thomson Scientific, London, GB; AN 2003-066054 XP002721407, & KR 2002 0059507 A (CJ CORP) 13. Juli 2002 (2002-07-13)
- SEI GOON O: "TOOTHPASTE COMPOSITION CONTAINING ESSENTIAL OIL FOR AROMA THERAPY AND MANUFACTURING PROCESS THEREOF", CA, 15. März 2001 (2001-03-15), XP002342203,
- "Reaction inhibitor for aroma-therapy - contg. rose oil, ethanol and propellant, used to relieve mental stress by aromatic therapy, in perfume, etc", DERWENT, 19. Juli 1991 (1991-07-19), XP002274343,
- ELIZABETH VARNEY ET AL: "Effect of Inhaled Essential Oils on Mental Exhaustion and Moderate Burnout: A Small Pilot Study", JOURNAL OF ALTERNATIVE AND COMPLEMENTARY MEDICINE, Bd. 19, Nr. 1, 1. Januar 2013 (2013-01-01) , Seiten 69-71, XP55106593, US ISSN: 1075-5535, DOI: 10.1089/acm.2012.0089
- PHILIPP B: "Der sanften Methode sind (fast) keine Grenzen gesetzt = Aroma therapy: there are hardly any limitations in this gentle method", PFLEGEZEITSCHRIFT, W. KOHLHAMMER GMBH, DE, Bd. 52, Nr. 10, 1. Oktober 1999 (1999-10-01), Seiten 696-699, XP009176778, ISSN: 0945-1129
- HONGRATANAWORAKIT T: "Aroma-therapeutic effects of massage blended essential oils on humans", NATURAL PRODUCT COMMUNICATIONS, NATURAL PRODUCT INC, US, Bd. 6, Nr. 8, 1. August 2011 (2011-08-01), Seiten 1199-1204, XP009176779, ISSN: 1934-578X

## Beschreibung

Die vorliegende Erfindung betrifft eine Zusammensetzung enthaltend ätherische Öle, die dazu geeignet ist, in der Aromatherapie als Arzneimittel eingesetzt zu werden. Ätherische Öle sind in organischen Lösungsmitteln lösliche Extrakte oder die organische Phase aus Wasserdampfdestillaten aus Pflanzen oder Pflanzenteilen, die einen starken, für die Herkunftspflanze charakteristischen Geruch haben. Ätherische Öle enthalten sekundäre Pflanzeninhaltsstoffe, die der Pflanze beispielsweise dazu dienen können, Insekten zur Bestäubung anzulocken, Schädlinge fernzuhalten oder sich gegen Krankheiten zu schützen, die z. B. durch Bakterien oder Pilze hervorgerufen werden.

Ätherische Öle bestehen grösstenteils aus Gemischen verschiedener Terpene, Sesquiterpene oder aromatischer Verbindungen. Sie sind also aus vielen verschiedenen chemischen Verbindungen zu zusammengesetzt. Ätherische Öle sind fettlöslich, enthalten jedoch keine Fette. Im Gegensatz zu fetten Ölen verdampfen ätherische Öle rückstandsfrei.

Ätherische Öle werden in Öldrüsen von Pflanzen gebildet und im Pflanzengewebe gespeichert. Sie befinden sich in Blüten, Blättern, Samen, Fruchtschalen, Wurzeln, Harzen, Rinden oder im Holz. Manche Pflanzen liefern aus verschiedenen Pflanzenteilen ätherische Öle, die sich in ihrer chemischen Zusammensetzung sehr stark unterscheiden, z. B. Zimtrinden- und Zimtblätteröl.

Das gebräuchlichste Verfahren zur Gewinnung von ätherischen Ölen aus Pflanzen ist die Wasserdampfdestillation. Öle einiger Blütenarten, wie Jasmin, Tuberose oder Mimose, können nicht per Wasserdampfdestillation gewonnen werden, und werden daher häufig mittels Extraktion isoliert. Als weiteres Verfahren ist die Kaltpressung bekannt, die nur für Zitrusöle (Agrumenöle) angewandt wird. Die sehr kostspielige Extraktion mit Fetten, die sogenannte Enfleurage, wird heute kaum mehr praktiziert.

Die Bezeichnung "ätherisches Öl" ist nicht geschützt und wird im Handel auch für rein synthetische Produkte verwendet. Zur Differenzierung der ätherische Öle werden die Bezeichnungen naturbelassen, natürlich, naturidentisch und künstlich verwendet:
- Naturbelassene Öle werden direkt aus Pflanzen gewonnen.
- Natürliche Öle bestehen aus mehreren naturreinen Komponenten, werden also nicht ausschließlich aus der namensgebenden Pflanze gewonnen. Natürliche Öle dürfen keine synthetischen Zusätze enthalten. Eine Mischung eines naturreinen Öles mit synthetischen Zusätzen bezeichnet man als natürlich/naturidentisch (N/NI).
- Die Bestandteile naturidentischer Öle werden nach dem Vorbild der chemischen Zusammensetzung natürlicher ätherischer Öle synthetisch hergestellt, so dass sie ähnlich wie natürliche Öle riechen. Die Zusammensetzung naturidentischer Öle ist häufig weniger komplex als die der natürlichen Varianten, so besteht beispielsweise naturidentisches Rosmarinöl aus ca. elf Bestandteilen, während das naturbelassene ätherische Öl ca. 150 Inhaltsstoffe hat.
- Künstliche Öle besitzen kein natürliches Gegenstück und werden gezielt auf bestimmte Geruchseigenschaften hin entworfen.

Ätherische Öle werden je nach Eigenschaft unterschiedlich genutzt. Häufig steht der Einsatz als Duftstoff in Kosmetik- und Parfümindustrie im Vordergrund, aber auch als medizinische Wirkstoffe und als technische Lösungsmittel haben bestimmte ätherische Öle Bedeutung.

Ätherische Öle unterliegen dem Arzneimittelrecht, sofern sie in Arzneimitteln enthalten sind. Sie sind im Deutschen Arzneibuch (DAB) sowie im Ph. Eur. (Europäischen Arzneimittelbuch) beschrieben und in über 2000 Medikamenten enthalten. Als Bestandteil von kosmetischen Präparaten unterliegen ätherische Öle den Regelungen der europäischen Kosmetikverordnung. In Lebensmittel und Bedarfsgegenständen unterliegen sie dem Lebensmittel-, Bedarfsgegenstände- und Futtermittelgesetzbuch. Ätherische Öle sind grundsätzlich frei verkäuflich und von jedem anwendbar.

Eine zentrale Rolle spielen ätherische Öle bei der naturheilkundlichen Methode der Aromatherapie, einer Form der Pflanzenheilkunde (Phytotherapie) zur ganzheitlich orientierten Behandlung von emotionalen Beschwerden und Erkrankungen, zur Prophylaxe und zur Gesunderhaltung mit Hilfe von Duftstoffen.

Die Aromatherapie bezeichnet die Anwendung ätherischer Öle als Aromaheilmittel zur Steigerung des Wohlbefindens. Sie darf in Deutschland von Ärzten und Heilpraktikern mit einer Zusatzqualifikation ausgeführt werden, da auch ätherische Öle unerwünschte Nebenwirkungen haben oder giftig sein können. Ebenfalls sind allergische Reaktionen möglich.

Duftstoffe allgemein können in verschiedenen Weisen auf den menschlichen Körper einwirken:
- Der Geruchssinn wird angesprochen; dies führt zu einer Sinneswahrnehmung mit all den damit verbundenen Nebeneffekten (Gefühlseindruck, Erinnerung, reflektorische Beeinflussung verschiedener Körperfunktionen, etc.).
- Nach Einnahme oder Inhalation können ätherische Öle auch eine direkte Wirkung auf die Organe haben.
- Einige ätherische Öle besitzen auch antibiotische Eigenschaften, wodurch sie sich gut für die Prophylaxe und zur Behandlung leichterer Infekte eignen.
- Es ist auch möglich, Öle direkt oder verdünnt auf die Haut aufzutragen.

Die Perzeption eines Geruchsreizes erfolgt in der Riechschleimhaut (*Regio olfactoria,* auch Riechepithel), einem ca. 2 x 2.5 cm² grossen Bereich an der oberen Nasenmuschel und im oberen Teil des Nasenseptums. Das Riechepithel setzt sich aus drei Zelltypen zusammen, den Sinnes-, Stütz- und Basalzellen. Der Mensch besitzt zwischen 10 und 100 Mio. olfaktorische Sensoren, die als Riechzellen bezeichnet werden und in das Riechepithel eingebettet sind. Diese Riechzellen sind bipolare Nervenzellen, deren kurze dendritischen Fortsätze in der Riechschleimhaut enden, wohingegen die langen axonalen Fortsätze zentral in den *Bulbus olfactorius* ziehen.

Duftmoleküle gelangen mit der Atemluft zur Riechschleimhaut. In der Membran der Riechzellen sitzt jeweils einer von insgesamt 350 verschiedenen Rezeptoren. Bindet sich ein Duftmolekül an einen Rezeptor, so sendet die Riechzelle ein Signal an einen der knopfartigen Glomeruli im Riechkolben. Der Riechkolben wiederum sendet das Signal an den olfaktorischen Cortex, wo der Reiz genauer analysiert wird.

Das Riechsystem des Menschen besitzt einen speziellen elektrischen Verstärkungsmechanismus, der die Riechzellen der Nase dazu befähigt, auch auf extrem schwache Reize zu reagieren. Wie Forscher herausfanden, bereiten sich die Sinneshärchen der Riechzelle in besonderer Weise auf ihren Einsatz vor: Ein Proteinkomplex pumpt Chloridionen in das Innere der Sinneshärchen, so dass diese zu gut gefüllten Chloridspeichern werden. Bei einem Duftreiz löst die schwache Reaktion der Duftstoffrezeptoren eine schlagartige Öffnung aller Chloridkanäle, die sich in der Aussenmembran des Sinneshärchens befinden, aus. Der Ausstrom negativ geladener Chloridionen verursacht eine Ladungsumkehrung der Riechzelle. Dadurch entstehen starke elektrische Signale, die mit den Geruchsinformationen zum Gehirn geleitet werden.

Einerseits wird der Riechreiz nun an den orbitofrontalen Cortex des Grosshirns weitergeleitet, wo er mit anderen Eindrücken, wie Geschmack, Tast-, Hör- oder Schmerzsinn, zu einer Gesamtempfindung zusammengesetzt wird. Vermutlich dringt die Wahrnehmung erst hier ins Bewusstsein. Andererseits werden auch Hypothalamus und Amygdala vom gerochenen Duft informiert. Die Amygdala verknüpft Düfte mit Emotionen und trägt so zur Bewertung des Geruchs bei. Der Hypothalamus steuert das Hormonsystem. Deshalb wird vermutet, dass Pheromone über diese Nervenbahn ihre Wirkung ausüben.

Die Zuflüsse zum limbischen System, die nur durch zwei Synapsen zwischen Riechepithel und Amygdala zustande kommen, erklären die enge Verbindung von Gerüchen und Gedächtnisprozessen und somit die emotionale Komponente der Duftstoffwahrnehmung. So können vergessene Erinnerungen durch die Wahrnehmung bestimmter Gerüche wieder ins Gedächtnis gerufen werden.

Für die Auslösung von emotionalen Reaktionen ist die rechte Hirnhälfte verantwortlich. Diese wird intensiver angesprochen von nonverbalen Reizen als von verbalen Botschaften. Zu den nonverbalen Reizen gehören unter anderem Farben, Bilder, Musik und Düfte. Entscheidend ist das Zusammenspiel verschiedener Umweltvariablen. Isolierte Effekte, sogenannte Einzelreize, wirken viel intensiver als Kombinationen und Bündelungen der Reize auf die Sinnesorgane.

Das Duftmolekül erzeugt im Riechepithel also ein elektrisches Signal, welches an die Nervenzellen - genauer die Synapse respektive den Synapsenspalt - geleitet wird.

Nervenzellen tauschen Informationen über diese speziellen Kontaktstellen, die Synapsen, aus. Ständig werden neue Synapsen aufgebaut, bestehende verstärkt und überflüssige abgebaut. Das Gehirn gleich einer Grossbaustelle. Ständig wachsen an der Oberfläche von Nervenzellen neue Fortsätze. Trifft ein Fortsatz auf die entsprechende Struktur einer Nachbarzelle, reifen die Fortsatzenden zu einer Synapse. Erst diese Kontaktstellen machen es möglich, Informationen von einer Zelle zur nächsten zu übertragen. Ist eine vorhandene Synapse ineffizient oder wird sie nicht mehr gebraucht, so wird sie wieder abgebaut.

In unserem Körper wimmelt es von Neuronen. Die Neuronen haben einen Zellkörper und ganz viele Verästelungen, die Dendriten und Axone. Axone können bis zu 1 m lang werden. Alle Dendriten besitzen ein "Endknöpfchen", die Synapse.

Das Grosshirn, wo die bewussten Informationen verarbeitet werden, umfasst allein etwa 14 Mrd. Neuronen. Jedes Neuron besitzt 1'000 bis 10'000 Synapsen, die wie ein Pelz um die Axone und Dendriten der anderen Neuronen wuchern, immer einen (synaptischen) Spalt freilassend. Die Wand dieser Hirnzellen ist eine durchgangsvariable Proteinmembran. Sie trennt K⁺-, Na⁺-, Cl⁻-Ionen und negative geladene Eiweissionen voneinander. Dadurch entsteht einen elektrische Spannung. In den Neuronen wird ständig ein Ruhepotential (elektrische Spannung) von 80 mV zwischen dem Zellinnern und der zellumgebenden Gewebeflüssigkeit aufrechterhalten. Bei Aktivierung eines Neurons ändert sich das Ruhepotential hin zum Aktionspotential, welches 30 mV in umgekehrter Polrichtung beträgt. Die Proteinmembran ändert dabei in Millisekundenbruchteilen ihre Durchlässigkeit (Porengrösse). Das Aktionspotential bleibt für eine Millisekunde bestehen und wird mit 120 m/s durch die Neuriten, Dendriten und Axone weitergeleitet. Die unzähligen Synapsen können das Aktionssignal nun durch den synaptischen Spalt in Form von Botenstoffen und Rezeptorproteinen in Bruchteilen von Millisekunden an andere Neuronen (Synapsen) weitergeben. Auch der elektrische Impuls, der durch die Duftmoleküle ausgelöst wird, wird als Aktionspotential an die entsprechenden Neuronen (Synapsen) im Gehirn geleitet und verarbeitet.

Man nimmt an, dass sich beim Auftreten einer belastenden Emotion der Synapsenspalt öffnet und mit einem elektrischen Gewitter mit negativ geladenen Signalen reagiert. Somit wird die Synapse aktiviert und ändert vom Ruhepotential hin zum Aktionspotential. Die Proteinmembran ändert dabei in Millisekundenbruchteilen ihre Durchlässigkeit (Porengrösse). Das Aktionspotential bleibt für eine Millisekunde bestehen und wird mit 120 m/s durch die Neuriten, Dendriten und Axone weitergeleitet. Die unzähligen Synapsen können das Aktionssignal nun durch den synaptischen Spalt in Form von Botenstoffen und Rezeptorproteinen in Bruchteilen von Millisekunden an andere Neuronen (Synapsen) weitergeben. Auch das elektrische Signal, das durch die Duftmoleküle ausgelöst wird, wird als Aktionspotential an die entsprechenden Neuronen (Synapsen) im Gehirn geleitet und verarbeitet.

Diese Reaktion ist in akuten Situationen absolut sinnvoll und manchmal sogar überlebenswichtig, wenn beispielsweise in einer Gefahrensituation eine schnelle Abwehrreaktion nötig ist. Allerdings können Menschen sich an solche schockierenden Erlebnisse noch lange danach immer wieder sehr genau erinnern und diese Situation "noch einmal durchleben", mitsamt aller emotionaler Folgen, was sehr belastend sein kann.

Es wird vermutet, dass solche belastenden Emotionen und Stresssymptome durch die im Körper immer noch festgehaltene und gebundene Energie im Nervensystem bedingt sind, welche ursprünglich durch physiologische und instinktive Mechanismen bereitgestellt wurde, um eine Angriffs- oder Fluchtreaktion zu ermöglichen. Das biologische System eines traumatisierten Menschen ist dabei immer aktiviert, auch wenn die Gefahr schon längst vorüber ist. Die negativen Informationen aus der belastenden Erfahrung sind auf den Synapsen gespeichert. Dies kann sich in einer Palette von Symptomen äussern, obwohl das ursprüngliche Objekt oder die Ursache nicht mehr bewusst wahrgenommen wird.

Aus solchen Beobachtungen wird ersichtlich, dass ein Schock oder Trauma nicht nur rein mit dem Verstand verarbeitet werden kann, sondern vor allem emotional und körperlich angegangen werden muss. Ist eine traumatische Erfahrung nicht mehr im Bewusstsein, so kann diese unter Beihilfe von ätherischen Ölen (Duftgedächtnis) und therapeutischer Hypnose oder Selbsthypnose aktiviert und aus dem Unterbewusstsein ins Bewusstsein geholt werden. Eine wirksame und vor allem langfristig anhaltende Therapie solcher belastender Emotionen ist bis anhin nur über langwierige therapeutische Interventionen, wenn überhaupt, möglich.

Es ist daher eine Aufgabe der vorliegenden Erfindung, ein einfaches und schnell anzuwendendes Mittel zur Behandlung von belastenden Emotionen und Stresssymptomen zur Verfügung zu stellen.

Diese Aufgabe wird durch die nachfolgend beschriebene Zusammensetzung sowie deren Verwendung gelöst.

Die erfindungsgemässe Zusammensetzung eignet sich zur Verwendung für die Aromatherapie, Aromaheilkunde und Aromatologie. Die Zusammensetzung umfasst eine Wirkzusammensetzung und eine Transportzusammensetzung. Optional umfasst sie weiter ein Trägeröl und/oder einen Alkohol.

Die Wirkzusammensetzung liegt erfindungsgemäss in einem Anteil von 0.01 bis 0.125 Volumenprozent vor, während die Transportzusammensetzung einen Anteil von 0.1 bis 99.99 Volumenprozent ausmacht.

Die Wirkzusammensetzung enthält ein Gemisch von 90 bis 110 verschiedenen ätherischen Ölen und einem Alkohol in einem Volumenverhältnis von 1:500 bis 1:5'000 Öle:Alkohol. Sie enthält also mit Alkohol stark verdünnte ätherische Öle. Der Verdünnungsgrad ist derart hoch, dass die ätherischen Öle der Wirkzusammensetzung für den Menschen üblicherweise nicht riechbar sind.

Die Transportzusammensetzung besteht aus einem oder mehreren ätherischen Ölen. Die Transportzusammensetzung ist in einer Konzentration vorhanden, in der sie für den Menschen riechbar ist.

Das Trägeröl, falls vorhanden, ist ausgewählt aus der Gruppe bestehend aus Avocadoöl, Callophyllumöl, Hanföl, Johanniskrautöl, Jojobaöl, Kokosfett, Macadamianussöl, Mandelöl, Nachtkerzenöl, Olivenöl, Ringelblumenöl, Sanddornöl, Schwarzkümmelöl, Sesamöl, Sheabutter, Traubenkernöl, Vitexöl, Wildrosenöl und Mischungen davon. Der Anteil an Trägeröl beträgt maximal 99.89 Volumenprozent.

Der in der Wirkzusammensetzung vorhandene und der optional zusätzlich in der erfindungsgemässen Zusammensetzung vorhandene Alkohol sind unabhängig voneinander ausgewählt aus der Gruppe bestehend aus Methanol, Ethanol, Propanol und Isopropanol. Vorzugsweise ist in beiden Fällen Ethanol enthalten. Der Anteil an Alkohol beträgt maximal 50 Volumenprozent.

Im Einklang mit der vorliegenden Erfindung sind die ätherischen Öle der Wirkzusammensetzung ausgewählt aus der Gruppe bestehend aus Salbei (Salvia officinalis); Schopflavendel (Lavandula stoechas L.); Baldrian (Valeriana officinalis); Iris (Iris pallida var. florentina); Myrrhe (Commiphora myrrha oder Commiphora molmol); Osmanthus (Osmanthus fragrans); Atlaszeder (Cedrus atlantica); Ingwer (Zingiber officinalis); Kamille, insbesondere echte blaue oder echte Kamille (Matricaria camomilla oder Chamomilla recutita); Manuka (Leptospermum scoparium); Melisse (Melissa officinalis CT Citral); Narde (Nardostachys jatamansi); Patchouli (Pogostemon cablin); Schafgarbe (Achillea millefolium L.); Vetiver (Vetiveria zizanoides); Virginia Wacholder (Juniperus virginiana L.); Ylang Ylang (Cananga odorata var. genuina); Karottensamen (Daucus carotta L.); Sandelholz (Santalum album oder Santalum austrocaledonicum); Basilikum Ct Linalool (Ocimum basilicum); Bergamottminze oder Zitronenminze (Mentha citrata L.); Geranie (Pelargonium graveolens); Koriandersamen (Coriandrum sativum L.); Linaloeholz (Bursera delpechiana); Neroli (Citrus aurantium var. amara); Palmarosa (Cymbopogon martinii); Pfefferminze (Mentha x piperita); Damaszener Rose (Rosa damascena); Rosenholz (Aniba parviflora oder Aniba rosaeodora); Speiklavendel (Lavandula spica oder Lavandula latifolia); Thymian CT Linalool (Thymus vulgaris oder Thymus officinalis); Thymian CT Thujanol (Thymus vulgaris CT Thujanol-4); Cajeput (Melaleuca cajeputi oder Melaleuca leucadendron); Eukalyptus globulus (Eucalyptus globulus); Eukalyptus radiata (Eucalyptus radiata); Kardamom (Elettaria cardamomum L.); Lorbeer (Laurus nobilis L.); Niaouli (Melaleuca viridiflora oder Melaleuca quinquinervia); Ravintsara (Cinnamomum camphora); Rosmarin CT Cineol (Rosmarinus officinalis); Thymian Mastichina (Thymus mastichina); Ysop decumbens (Hyssopus officinalis var. decumbens); Eisenkraut oder Verbena oder Zitronenstrauch (Lippia citriodora Kuntze); Lemongrass oder Zitronengras (Cymbopogon flexuosus); Litsea (Litsea cubeba); Zitroneneukalyptus (Eucalyptus citriodoria); Angelikawurz oder Engelwurz (Angelica archangelica); Bergamotte (Citrus aurantium var. bergamia); Cistrose (Cistus ladaniferus L.); Douglasie (Pseudotsuga menziesii); Elemi (Canarium luzonicum A.); Estragon (Artemisia dracunculus L.); sibirische Fichte (Abies sibirica); Galbanum (Ferula galbaniflua); Grapefruit (Citrus paradisi); Kiefer oder Waldföhre (Pinus sylvestris); Kreuzkümmel (Cuminum cyminum L.); Latschenkiefer (Pinus mugo); Limette (Citrus aurantifolia); Majoran (Origanum majorana oder Majorana hortensis); rote Mandarine (Citrus madurensis oder Citrus reticulata); Anden-Myrte (Myrtus communis); Marokko-Myrte (Myrtus communis CT Myrtenylacetat); Orange (Citrus sinensis); schwarzer Pfeffer (Piper nigrum); Riesentanne (Abies grandis L.); Rosmarin CT Verbenon (Rosmarinus officinalis); Teebaum (Melaleuca alternifolia); Wacholderbeere (Juniperus communis); arabischer Weihrauch oder Olibanum (Boswellia Carterii); Zedrat oder Ur-Zitrone (Citrus medica); Zitrone (Citrus limon(um)); Zypresse (Cupressus sempervirens); Thymian CT Thymol (Thymus vulgaris oder Thymus officinalis); Cassia (Cinnamomum cassia oder Cinnamonum aromaticum); Nelkenknospen (Syzygium aromaticum oder Eugenia caryophyllus); Tulsi oder heiliges Basilikum (Ocimum sanctum L.); Zimtblatt (Cinnamomum ceylanicum); Anissamen (Pimpinella anisum); Fenchel, insbesondere süsser Fenchel (Foeniculum vulgare var. dulce); Tonka (Dipteryx odorata); Immortelle (Helichrysum italicum G.); Kamille, insbesondere römische Kamille (Anthemis nobilis oder Chamaemelum nobile); Lavandin (Lavandula hybrida); Lavendel (Lavandula officinalis oder Lavandula vera oder Lavandula angustifolia); Muskatellersalbei (Salvia sclarea); Petit grain Bitterorange (Citrus aurantium L. var. pumilia); Eritrea-Weihrauch (Boswellia carterii); Zuckerbirke (Betula lenta); Vanille (Vanilla planifolia); Siam-Benzoe (Styrax tonkinensis oder Styrax benzoin); Champaca (Michelia champaca L.); Ginster (Spartium junceum L.); Sambac-Jasmin (Jasminum sambac); Petit grain Mandarine (Citrus reticulata blanco); Wintergrün (Gaultheria fragrantissima); und Ylang Ylang (Cananga odorata var. genuina).

Besonders bevorzugt umfasst die Wirkzusammensetzung alle oder im Wesentlichen alle vorstehend genannten ätherischen Öle.

Ferner sind die ätherischen Öle der Transportzusammensetzung erfindungsgemäss ausgewählt aus der Gruppe bestehend aus Vetiveria zizanoides, Daucus carotta L., Nardostachys jatamansi, Santalum album, Santalum austrocaledonicum, Cananga odorata var. genuina, Elettaria cardamomum L., Citrus paradisi, Cymbopogon flexuosus, Litsea Cubeba, Pelargonium graveolens, Melissa officinalis CT Citral, Rosa damascena, Commiphora myrrha, Commiphora molmol, Salvia officinalis, Melaleuca cajeputi, Melaleuca leucadendron, Hyssopus officinalis var. decumbens, Canarium luzonicum A., Ferula galbaniflua, Boswellia Carterii, Cedrus atlantica und Citrus limonum.

Die erfindungsgemässe Zusammensetzung wirkt lösend, befreiend, harmonisierend, vitalisierend, erleichternd, erdend, klärend, entspannend, stressabbauend, nervenstärkend, tröstend, ausgleichend, stimmungshebend, stabilisierend, beruhigend.

Vor allem hat sich aber gezeigt, dass mit Hilfe der erfindungsgemässen Zusammensetzung belastende Emotionen vollständig abgelöst werden können.

Dazu werden die emotionalen Trigger reaktiviert, beispielsweise indem sich der Betroffene die belastende Situation vorstellt, mittels eines Duftreizes oder durch Hypnose, so dass die belastende Erfahrung wieder aus dem Unterbewusstsein ins Bewusstsein geholt wird. Dabei wird der Betroffene üblicherweise starke (belastende) Emotionen verspüren.

Indem der Betroffene aber nun an der erfindungsgemässen Zusammensetzung riecht, sobald er diese Emotionen verspürt, gelangen die Duftmoleküle der Zusammensetzung und die daraus resultierenden elektrischen Impulse an die zur Negativerfahrung korrespondierende Synapsenkette und lösen die als negativ empfundenen Emotionen nachhaltig ab. Diese belastenden Emotionen können auch zu einem späteren Zeitpunkt nicht mehr abgerufen werden.

Es wird vermutet, dass positive elektrische Impulse (Duftmoleküle) auf negative elektrische Impulse (belastende Emotion) an der Synapse treffen, wobei sich die Impulse gegenseitig neutralisieren. Dadurch wird das belastende Gefühl nachhaltig abgelöst. Somit wird durch das erneute Hervorrufen der belastenden Emotion und das gleichzeitige Riechen an der erfindungsgemässen Zusammensetzung diese Emotion nachhaltig abgelöst.

Die Anwendung der erfindungsgemässen Zusammensetzung ist sehr unkompliziert und kostengünstig: Durch einfaches Riechen an der Zusammensetzung wird ein positiver Effekt erzielt. Es sind keine unerwünschten Nebenwirkungen bekannt, auch nicht mit Medikamenten. Zudem besteht auch keine Gefahr einer Überdosierung.

Die Behandlung mit der erfindungsgemässen Zusammensetzung leitet einen ganzheitlichen Verarbeitungsprozess ein. Es werden dabei keine Symptome unterdrückt. Ausserdem kann der Anwender völlig frei bestimmen, wann und wo er welche Themen ablösen möchte. Damit liegt auch das Tempo der Behandlung vollständig in seiner Bestimmung.

Es hat sich gezeigt, dass der Verdünnungsgrad der Wirkzusammensetzung für diesen Effekt eine sehr entscheidende Rolle spielt. So waren Versuche mit einer deutlich höheren Konzentration an ätherischen Ölen nicht erfolgreich. Ebenso scheint eine gewisse Mindestkonzentration nötig zu sein. Die Wirksamkeit der erfindungsgemässen Zusammensetzung auf der Bewusstseinsebene wurde durch positive multizentrische Verlaufsbeobachtungen belegt.

Es wird vermutet, dass die Transportzusammensetzung es erst möglich macht, dass das elektrische Signal der erfindungsgemässen Zusammensetzung überhaupt an die Nervenzellen und Synapsen gelangen kann und somit eine Signalübertragung stattfinden kann.

Gemäss einer bevorzugten Ausführungsform enthält die Wirkzusammensetzung ätherische Öle und Alkohol in einem Volumenverhältnis von 1:1'000 bis 1:2'000. Vorzugsweise beträgt das Volumenverhältnis Öle: Alkohol 1:1'300 bis 1:1'400, besonders bevorzugt in einem Volumenverhältnis von 1:1'370 bis 1:1'380.

Die besten Resultate wurden mit einem Volumenverhältnis von 1:1'378 ätherische Öle zu Alkohol in der Wirkzusammensetzung erzielt. Insbesondere wurde hier eine nachhaltige Ablösung der belastenden Emotion beobachtet.

Gemäss einer bevorzugten Ausführungsform enthält die Wirkzusammensetzung ein Gemisch von 90 bis 110, vorzugsweise von 95 bis 100 und besonders bevorzugt von 98 verschiedenen ätherischen Ölen. Es hat sich gezeigt, dass durch ein solches Gemisch eine ganzheitliche Behandlung einer grossen Vielfalt von belastenden Emotionen möglich ist. Insbesondere können Traumata, Schock, Stressfolgeerkrankungen, psychischen und somatischen Beschwerden, Angespanntheit, Angst, Antriebslosigkeit, Einsamkeit, emotionalem Schmerz, Erschöpfung, Nervosität, Ohnmacht, Trauer, Überforderung, Unruhe, Verzweiflung und/oder Wut gelöst werden.

Vorzugsweise umfassen die Wirkzusammensetzung jeweils mindestens ein ätherisches Öl, das geistig ausgleichend, öffnend, geistig entspannend, beruhigend, ganzheitlich entspannend, körperlich entspannend, erdend, körperlich ausgleichend, körperlich anregend, vitalisierend, ganzheitlich anregend, geistig anregend und stimulierend wirkt.

Vorzugsweise umfassen die in der Wirkzusammensetzung enthaltenen ätherischen Öle Monoterpene, Monoterpenketone, Monotherpenaldehyde, Monoterpenalkohole, Monoterpenphenole, Monoterpenoxide, Monoterpenether, Monoterpenester, Sesquiterpene, Sesquiterpenketone, Sesquiterpenaldehyde, Sesquiterpenalkohole, Sesquiterpenoxide, Diterpenalkohole, aromatische Aldehyde, aromatische Ketone, aromatische Säuren, aromatische Ester, aromatische Alkohole, Cumarine, Furocumarine, Eugenol und Zimtaldehyd.

Vorzugsweise enthält die Wirkzusammensetzung ausschliesslich natürliche ätherische Öle.

Gemäss einer bevorzugten Ausführungsform umfasst die erfindungsgemässe Zusammensetzung 0.04 bis 0.07

Volumenprozent an Wirkzusammensetzung, besonders bevorzugt 0.06 Volumenprozent.

Grundsätzlich kann die Transportzusammensetzung aus einem einzelnen ätherischen Öl bestehen.

Bevorzugt besteht die Transportzusammensetzung aber aus einem Gemisch von 7 bis 35 verschiedenen ätherischen Ölen, besonders bevorzugt aus 14 bis 28 verschiedenen ätherischen Ölen, und insbesondere aus 21 verschiedenen ätherischen Ölen. Diese Öle liegen vorzugsweise in gleichen Volumenanteilen vor, wobei aber auch andere Mischverhältnisse möglich sind.

Gemäss einer besonders bevorzugten Ausführungsform besteht die Transportzusammensetzung aus ätherischen Ölen von Vetiver (Vetiveria zizanoides), Karottensamen (Daucus carotta), Narde (Nardostachys jatamansi), Sandelholz (Santalum album), Ylang Ylang komplett (Cananga odorata), Kardamom (Elettaria cardamomum), Grapefruit (Citrus paradisi), Lemongrass (Cymbopogon flexuosus), Litsea Cubeba (Litsea cubeba), Geranium (Pelargonium graveolens), Melisse (Melissa officinalis), Rose (Rosa damascena), Myrrhe (Commiphora myrrha), Salbei (Salvia officinalis), Cajeput (Melaleuca cajeputi), Ysop (Hyssopus officinalis), Elemi (Canarium luzonicum), Galbanum (Ferula galbaniflua), Weihrauch (Boswellia carterii), Atlaszeder (Cedrus atlantica) und Zitrone (Citrus limon). Vorzugsweise liegen diese Öle in gleichen Volumenanteilen vor.

Die oben genannten 21 ätherischen Öle enthalten üblicherweise:

| | |
|---|---|
| Vetiver | 45-50% Vetivene, 35% Vetiverol/Khusimol, 15% Vetivone/Vetiveron/Khusimon |
| Karottensamen | 50-60% Carotol Daucol, 15-25% Pinene, 10-20% β-Bisabolen, 2-5% Linaool, 3% Geranylacetat |
| Narde | 60-66% Patchoulene/Gurjunen, 8-15% Valeranon/β-Jonon, 6-8% Patchoulialkohol/Valerianol |
| Sandelholz | 85-95% Santalole, 5-10% Santalene |
| Ylang Ylang | 55-70% Germacren/β-Caryophyl, 10-20% Benzylbenzoat/Benzylacetat, 10-20% Limalool, 7-15% p-Cresol-Methylether, 12% Geranylacetat |
| Kardamom | 35-50% 1,8-Cineol, 32-45% Terpinylacetat |
| Grapefruit | 90-98% (1)-Limonen (Merkaptan in Spuren), 0.5-1.8% Nootkaton |
| Lemongrass | 70-85% Citral, 5-10% Limonen, bis 10% Farnesol, bis 6% Geraniol, 3% Farnesal |
| Litsea Cubeba | 70-80% Citral, 10-15% Limonen, 5-10% Linalool/Geraniol/Nerol, bis 4.5% Methylheptenon |
| Geranium | 50-65% Citronellol/Geraniol, 15-30% Geranylacetat, 5-10% iso-Menthon, 5% Citral, 3-5% Rosenoxide |
| Melisse | 40-60% β-Caryophyllen (bis 30%ig), 25-55% Citral |
| Rose | 65-75% Citronellol/Geraniol, bis 4% Citronellylacetat/Geranylacetat, 2-3% Phenylethylalkohol, 2-3% Methyleugenol, bis 1% Rosenoxide, bis 1% Rosenketone |
| Myrrhe | Bis 60% Furanosesquiterpene, 20-45% Elemene/Copaen |
| Salbei | 30-60% Thuyon, 8-15% 1,8-Cineol, 5-15% Pinene, 5-10% Borneol |
| Cajeput | 50-65% 1,8-Cineol, 25-40% Pinene, 6-15% α-Terpineol |
| Ysop | 40-60% 1,8-Cineol, 20-30% β-Pinen, 6% Isopinocamphon |
| Elemi | 70-80% Limonen (bis 55%ig), 15-17% Elemol (bis 16%ig), 3-6% Elemicin |
| Galbanum | 45.1-58.8% β-Pinen, 2.0-12.1% 3-Caren, 5.7-12.0% α-Pinen, bis 6.4% Sabinen, bis 4.6% β-Myrcen, 2.7-4.0% (+)-Limonen, bis 2.4% Elemen, 1.6-1.8% 1,3,5-Undecatrien, bis 1.2% Ocimen |
| Weihrauch | 55% Octylacetat, 8% Octanal, 2.5% Incensol |
| Atlaszeder | 75-80% Himachalen, 3-15% Himachalol, 3-12% Atlanton, 1% Himachalenoxid |
| Zitrone | 80-95% (+)-Limonen (60-80%ig), 3-10% Citral |

Vorzugsweise enthält die Transportzusammensetzung ausschliesslich natürliche ätherische Öle.

Gemäss einer bevorzugten Ausführungsform umfasst die erfindungsgemässe Zusammensetzung 0.5 bis 20 Volumenprozent an Transportzusammensetzung, vorzugsweise 1 bis 5 Volumenprozent, besonders bevorzugt 2.5 bis 3 Volumenprozent, und insbesondere 2.94 Volumenprozent.

Niedrige Anteile an Transportzusammensetzung haben insbesondere den Vorteil, dass Zusammensetzungen mit einem maximalen Gehalt an ätherischen Ölen von 3% als Kosmetika gelten. Damit unterliegen sie nicht dem Heilmittelgesetz und es ist keine entsprechende Zulassung erforderlich. Ausserdem wird dadurch auch die Geruchsintensivität der erfindungsgemässen Zusammensetzung etwas verringert.

Besonders bevorzugt enthält die erfindungsgemässe Zusammensetzung daher 0.06 Volumenprozent der Wirkzusammensetzung und 2.94 Volumenprozent der Transportzusammensetzung. Die restlichen Anteile werden bevorzugt durch ein Trägeröl gebildet, wobei auch noch Alkohol oder weitere Bestandteile enthalten sein können.

Gemäss einer bevorzugten Ausführungsform enthalten die Wirkzusammensetzung und die Transportzusammensetzung ausschliesslich natürliche ätherische Öle, besonders bevorzugt solche aus biologischen Verfahren.

Gemäss einer bevorzugten Ausführungsform umfasst die erfindungsgemässe Zusammensetzung mindestens 50 Volumenprozent an Trägeröl, vorzugsweise mindestens 80 Volumenprozent, besonders bevorzugt mindestens 95 Volumenprozent, insbesondere mindestens 97 Volumenprozent, wobei der Anteil an Trägeröl maximal 99.89 Volumenprozent. Dadurch kann insbesondere erreicht werden, dass die erfindungsgemässe Zusammensetzung als Kosmetikum gilt.

Alternativ ist es auch möglich, dass die erfindungsgemässe Zusammensetzung weniger oder gar kein Trägeröl enthält. Insbesondere kann die Zusammensetzung auch dazu bestimmt sein, vor der Anwendung weiter verdünnt zu werden, beispielsweise mit einem Trägeröl.

In einem weiteren Aspekt betrifft die vorliegende Erfindung auch die Verwendung der erfindungsgemässen Zusammensetzung als Arzneimittel.

Die erfindungsgemässe Zusammensetzung ist insbesondere dazu geeignet, emotionale Beschwerden zu behandeln und zum allgemeinen Wohlbefinden beizutragen.

Besonders gut eignet sich die Zusammensetzung zur Behandlung von Traumata, Schock, Stressfolgeerkrankungen, psychischen und somatischen Beschwerden, Angespanntheit, Angst, Antriebslosigkeit, Einsamkeit, emotionalem Schmerz, Erschöpfung, Nervosität, Ohnmacht, Trauer, Überforderung, Unruhe, Verzweiflung und/oder Wut.

Die erfindungsgemässe Zusammensetzung zur Verwendung in der Aromatherapie hat sich insbesondere bei der Behandlung von emotionalen Beschwerden, wie Traumata, Schock, Stressfolgeerkrankungen, psychischen und somatischen Beschwerden, Angespanntheit, Angst, Antriebslosigkeit, Einsamkeit, emotionalem Schmerz, Erschöpfung, Nervosität, Ohnmacht, Trauer, Überforderung, Unruhe, Verzweiflung und/oder Wut, bewährt.

Die vorliegende Erfindung wird anhand der nachfolgenden Beispiele weiter verdeutlich, wobei diese nicht als einschränkend anzusehen sind:
Beispiel einer erfindungsgemässen Zusammensetzung
   0.06 Vol-% Wirkzusammensetzung
   2.94 Vol-% Transportzusammensetzung
   97.00 Vol-% Trägeröl
Wirkzusammensetzung: 98 ätherische Öle zu gleichen Anteilen aus Salbei (Salvia officinalis), Schopflavendel (Lavandula stoechas L.), Baldrian (Valeriana officinalis), Iris (Iris pallida var. florentina), Myrrhe (Commiphora myrrha / molmol), Osmanthus 5% (Osmanthus fragrans), Atlaszeder (Cedrus atlantica), Ingwer (Zingiber officinalis), Kamille blau (echte Kamille) (Matricaria camomilla / Chamomilla recutita), Manuka (Leptospermum scoparium), Melisse 10% (Melissa officinalis CT Citral), Narde (Nardostachys jatamansi), Patchouli (Pogostemon cablin), Schafgarbe (Achillea millefolium L.), Vetiver (Vetiveria zizanoides), Wacholder, Virginia (Juniperus virginiana L.), Ylang Ylang komplett (Cananga odorata var. genuina), Karottensamen (Daucus carotta L.), Sandelholz (Santalum album / S. austrocaledonicum), Basilikum Ct Linalool (Ocimum basilicum), Bergamottminze / Zitronenminze (Mentha citrata L.), Geranie (Rosen-) (Pelargonium graveolens), Koriandersamen (Coriandrum sativum L.), Linaloeholz (Bursera delpechiana), Neroli (Citrus aurantium var. amara), Palmarosa (Cymbopogon martinii), Pfefferminze (Mentha x piperita), Rose Damaszener 10% (Rosa damascena), Rosenholz (Aniba parviflora / rosaeodora), Speiklavendel (Lavandula spica / Lavandula latifolia), Thymian CT Linalool (Thymus vulgaris / officinalis), Thymian CT Thujanol (Thymus vulgaris CT Thujanol-4), Cajeput (Melaleuca cajeputi / M. leucadendron), Eukalyptus globulus (Eucalyptus globulus), Eukalyptus radiata (Eucalyptus radiata), Kardamom (Elettaria cardamomum L.), Lorbeer (Laurus nobilis L.), Niaouli (Melaleuca viridiflora / Melaleuca quinquinervia), Ravintsara (Cinnamomum camphora), Rosmarin CT Cineol (Rosmarinus officinalis), Thymian Mastichina (Thymus mastichina), Ysop decumbens (Hyssopus officinalis var. decumbens), Eisenkraut / Verbena / Zitronenstrauch (Lippia citriodora Kuntze), Lemongrass / Zitronengras (Cymbopogon flexuosus), Litsea (Litsea cubeba), Zitroneneukalyptus (Eucalyptus citriodoria), Angelika-/Engelwurz (Angelica archangelica), Bergamotte (Citrus aurantium var. bergamia), Cistrose, (Cistus ladaniferus L.), Douglasie (Pseudotsuga menziesii), Elemi (Canarium luzonicum A.), Estragon (Artemisia dracunculus L.), Fichte sibirisch (Abies sibirica), Galbanum (Ferula galbaniflua), Grapefruit (Citrus paradisi), Kiefer (Waldföhre) (Pinus sylvestris), Kreuzkümmel (Cuminum cyminum L.), Latschenkiefer (Pinus mugo), Limette (Citrus aurantifolia), Majoran (Origanum majorana / Majorana hortensis), Mandarine rot (Citrus madurensis / Citrus reticulata), Myrte Anden (Myrtus communis), Myrte Marokko (Myrtus communis CT Myrtenylacetat), Orange süss (Citrus sinensis), Pfeffer schwarz (Piper nigrum), Riesentanne (Abies grandis L.), Rosmarin CT Verbenon (Rosmarinus officinalis), Teebaum (Melaleuca alternifolia), Wacholderbeere (Juniperus communis), Weihrauch (Olibanum) arabisch (Boswellia Carterii), Zedrat / Ur-Zitrone (Citrus medica), Zitrone (Citrus limon(um)), Zypresse (Cupressus sempervirens), Thymian CT Thymol (Thymus vulgaris / officinalis), Cassia (Cinnamomum cassia / aromaticum), Nelkenknospen (Syzygium aromaticum / Eugenia caryophyllus), Tulsi / heiliges Basilikum (Ocimum sanctum L.), Zimtblatt (Cinnamomum ceylanicum), Anissamen (Pimpinella anisum), Fenchel süss (Foeniculum vulgare var. dulce), Tonka (Dipteryx odorata), Immortelle (Helichrysum italicum G.), Kamille römisch (Anthemis nobilis / Chamaemelum nobile), Lavandin (Lavandula hybrida), Lavendel fein (Lavandula officinalis / vera / angustifolia), Muskatellersalbei (Salvia sclarea), Petit grain Bitterorange (Citrus aurantium L. var. pumilia), Weihrauch Eritrea (Boswellia carterii), Vanille (Vanilla planifolia), Benzoe Siam (Styrax tonkinensis / Styrax benzoin), Champaca (Michelia champaca L.), Ginster (Spartium junceum L.), Jasmin sambac (Jasminum sambac), Petit grain Mandarine (Citrus reticulata blanco), Wintergrün (Gaultheria fragrantissima), und Ylang Ylang extra (Cananga odorata var. genuina); verdünnt mit 96% Ethanol (potabile) in einem Verhältnis von ätherischen Ölen zu Ethanol von 1:1'378.
Transportzusammensetzung: 21 ätherische Öle zu gleichen Anteilen aus Vetiver (Vetiveria zizanoides), Karottensamen (Daucus carotta), Narde (Nardostachys jatamansi), Sandelholz (Santalum album), Ylang Ylang komplett (Cananga odorata), Kardamom (Elettaria cardamomum), Grapefruit (Citrus paradisi), Lemongrass (Cymbopogon flexuosus), Litsea Cubeba (Litsea cubeba), Geranium (Pelargonium graveolens), Melisse (Melissa officinalis), Rose (Rosa damascena), Myrrhe (Commiphora myrrha), Salbei (Salvia officinalis), Cajeput (Melaleuca cajeputi), Ysop (Hyssopus officinalis), Elemi (Canarium luzonicum), Galbanum (Ferula galbaniflua), Weihrauch (Boswellia carterii), Atlaszeder (Cedrus atlantica) und Zitrone (Citrus limon).

### Multizentrische Verlaufsbeobachtung im Bewusstseinsstadium mit der erfindungsgemässen Zusammensetzung

Durch Therapeuten, Ärzte, Psychologen angeleitet.

Zeitraum: 17. März 2013 - 26. November 2013
Auswertungen aus 305 Erhebungen (Stand November 2013) auf der Skala 0 - 10 (0=keine Emotion, 10=extrem starke Emotion)

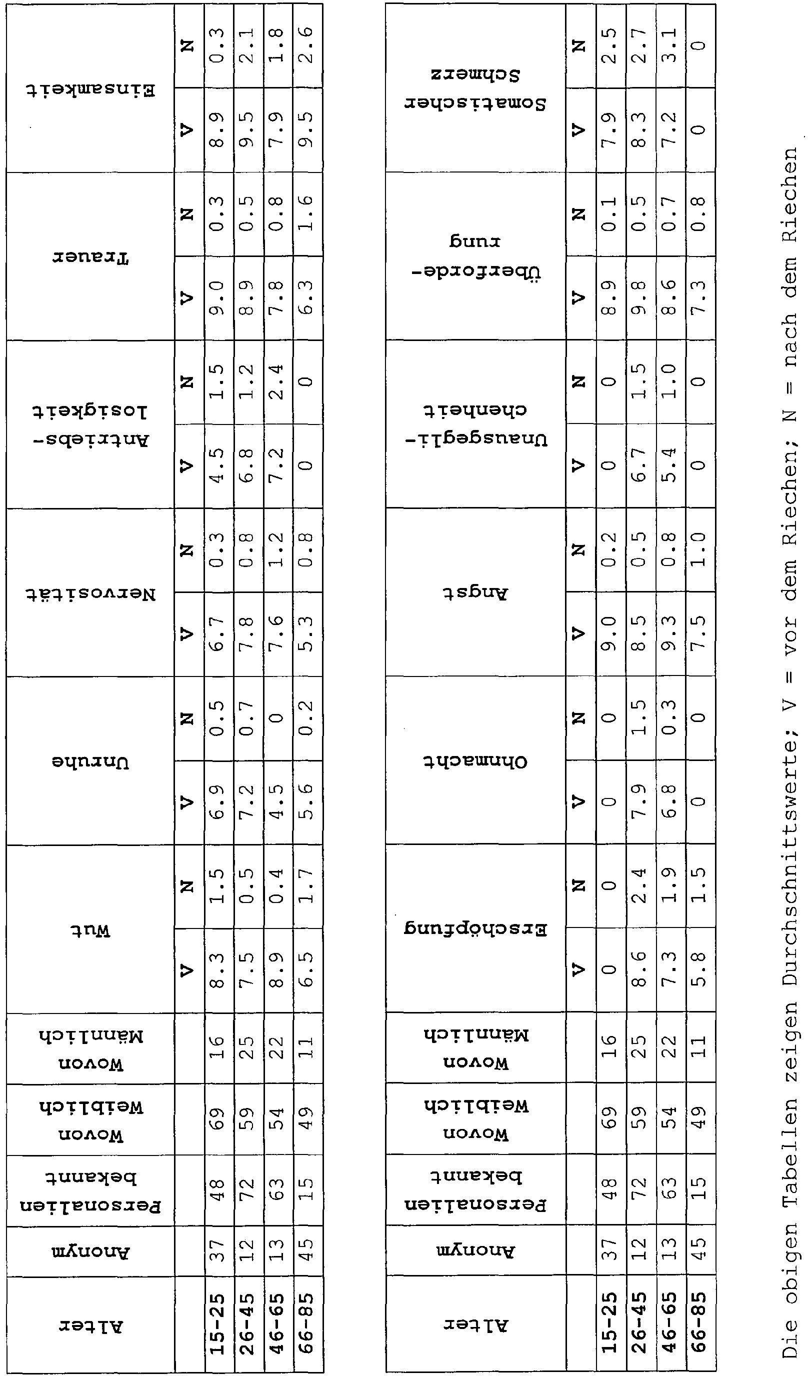

Die bisherigen Auswertungen zeigen eine signifikante Reduzierung der belastenden Emotionen nach dem intensiven Riechen an der erfindungsgemässen Zusammensetzung innert 1 Minute. Die besten Resultate wurden bei Wut, Angst und Trauer erzielt. Zudem wurde festgestellt, dass die belastende Emotion zur Ursprungserfahrung oftmals nach dem Riechen an der erfindungsgemässen Zusammensetzung gar nicht mehr abgerufen werden können, auch zu einem späteren Zeitpunkt nicht.

## Patentansprüche

1. Zusammensetzung zur Verwendung für die Aromatherapie, umfassend 0.01 bis 0.125 Volumenprozent einer Wirkzusammensetzung und 0.1 bis 99.99 Volumenprozent einer Transportzusammensetzung sowie optional ein Trägeröl und/oder einen Alkohol, wobei
die Wirkzusammensetzung ein Gemisch von 90 bis 110 verschiedenen ätherischen Ölen und einem Alkohol in einem Volumenverhältnis von 1:500 bis 1:5'000 Öle:Alkohol enthält,
die Transportzusammensetzung aus einem oder mehreren ätherischen Ölen besteht,
das Trägeröl ausgewählt ist aus der Gruppe bestehend aus Avocadoöl, Callophyllumöl, Hanföl, Johanniskrautöl, Jojobaöl, Kokosfett, Macadamianuss-öl, Mandelöl, Nachtkerzenöl, Olivenöl, Ringelblumen-öl, Sanddornöl, Schwarzkümmelöl, Sesamöl, Sheabutter, Traubenkernöl, Vitexöl, Wildrosenöl und Mischungen davon,
der Alkohol ausgewählt ist aus der Gruppe bestehend aus Methanol, Ethanol, Propanol und Isopropanol, vorzugsweise Ethanol,
wobei die ätherischen Öle der Wirkzusammensetzung ausgewählt sind aus der Gruppe bestehend aus Salvia officinalis, Lavandula stoechas L., Valeriana officinalis, Iris pallida var. florentina, Commiphora myrrha, Commiphora molmol, Osmanthus fragrans, Cedrus atlantica, Zingiber officinalis, Matricaria camomilla, Chamomilla recutita, Leptospermum scoparium, Melissa officinalis CT Citral, Nardostachys jatamansi, Pogostemon cablin, Achillea millefolium L., Vetiveria zizanoides, Juniperus virginiana L., Cananga odorata var. genuina, Daucus carotta L., Santalum album, Santalum austrocaledonicum, Ocimum basilicum, Mentha citrata L., Pelargonium graveolens, Coriandrum sativum L., Bursera delpechiana, Citrus aurantium var. amara, Cymbopogon martinii, Mentha x piperita, Rosa damascena, Aniba parviflora, Aniba rosaeodora, Lavandula spica, Lavandula latifolia, Thymus vulgaris, Thymus officinalis, Thymus vulgaris CT Thujanol-4, Melaleuca cajeputi, Melaleuca leucadendron, Eucalyptus globulus, Eucalyptus radiata, Elettaria cardamomum L., Laurus nobilis L., Melaleuca viridiflora, Melaleuca quinquinervia, Cinnamomum camphora, Rosmarinus officinalis, Thymus mastichina, Hyssopus officinalis var. decumbens, Lippia citriodora Kuntze, Cymbopogon flexuosus, Litsea cubeba, Eucalyptus citriodoria, Angelica archangelica, Citrus aurantium var. bergamia, Cistus ladaniferus L., Pseudotsuga menziesii, Canarium luzonicum A., Artemisia dracunculus L., Abies sibirica, Ferula galbaniflua, Citrus paradisi, Pinus sylvestris, Cuminum cyminum L., Pinus mugo, Citrus aurantifolia, Origanum majorana, Majorana hortensis, Citrus madurensis, Citrus reticulata, Myrtus communis, Myrtus communis CT Myrtenylacetat, Citrus sinensis, Piper nigrum, Abies grandis L., Rosmarinus officinalis, Melaleuca alternifolia, Juniperus communis, Boswellia Carterii, Citrus medica, Citrus limonum, Cupressus sempervirens, Thymus vulgaris, Thymus officinalis, Cinnamomum cassia, Cinnamonum aromaticum, Syzygium aromaticum, Eugenia caryophyllus, Ocimum sanctum L., Cinnamomum ceylanicum, Pimpinella anisum, Foeniculum vulgare var. dulce, Dipteryx odorata, Helichrysum italicum G., Anthemis nobilis, Chamaemelum nobile, Lavandula hybrida, Lavandula officinalis, Lavandula verä, Lavandula angustifolia, Salvia sclarea, Citrus aurantium L. var. pumilia, Boswellia carterii, Betula lenta, Vanilla planifolia, Styrax tonkinensis, Styrax benzoin, Michelia champaca L., Spartium junceum L., Jasminum sambac, Citrus reticulata blanco, Gaultheria fragrantissima, und Cananga odorata var. genuina, und
wobei die ätherischen Öle der Transportzusammensetzung ausgewählt sind aus der Gruppe bestehend aus Vetiveria zizanoides, Daucus carotta L., Nardostachys jatamansi, Santalum album, Santalum austrocaledonicum, Cananga odorata var. genuina, Elettaria cardamomum L., Citrus paradisi, Cymbopogon flexuosus, Litsea Cubeba, Pelargonium graveolens, Melissa officinalis CT Citral, Rosa damascena, Commiphora myrrha, Commiphora molmol, Salvia officinalis, Melaleuca cajeputi, Melaleuca leucadendron, Hyssopus officinalis var. decumbens, Canarium luzonicum A., Ferula galbaniflua, Boswellia Carterii, Cedrus atlantica und Citrus limonum.

2. Zusammensetzung zur Verwendung nach Anspruch 1, wobei die Wirkzusammensetzung ätherische Öle und Alkohol in einem Volumenverhältnis von 1:1'000 bis 1:2'000, vorzugsweise in einem Volumenverhältnis von 1:1'300 bis 1:1'400 und besonders bevorzugt in einem Volumenverhältnis von 1:1'370 bis 1:1'380, insbesondere in einem Volumenverhältnis von 1:1'378, enthält.

3. Zusammensetzung zur Verwendung nach Anspruch 1 oder 2, wobei die Wirkzusammensetzung ein Gemisch von 90 bis 110, vorzugsweise von 95 bis 100 und besonders bevorzugt von 98 verschiedenen ätherischen Ölen enthält.

4. Zusammensetzung zur Verwendung nach einem der Ansprüche 1 bis 3, umfassend 0.04 bis 0.07 Volumenprozent an Wirkzusammensetzung, bevorzugt 0.06 Volumenprozent.

5. Zusammensetzung zur Verwendung nach einem der Ansprüche 1 bis 4, wobei die Transportzusammensetzung aus einem Gemisch von 7 bis 35 verschiedenen ätherischen Ölen, bevorzugt aus 14 bis 28 verschiedenen ätherischen Ölen, und insbesondere aus 21 verschiedenen ätherischen Ölen, besteht, wobei die ätherischen Öle jeweils in gleichen Volumenanteilen vorliegen.

6. Zusammensetzung zur Verwendung nach einem der Ansprüche 1 bis 5, umfassend 0.5 bis 20 Volumenprozent an Transportzusammensetzung, vorzugsweise 1 bis 5 Volumenprozent, besonders bevorzugt 2.5 bis 3 Volumenprozent, und insbesondere 2.94 Volumenprozent.

7. Zusammensetzung zur Verwendung nach einem der Ansprüche 1 bis 6, wobei die Wirkzusammensetzung und die Transport-zusammensetzung ausschliesslich natürliche ätherische Öle enthalten.

8. Zusammensetzung zur Verwendung nach einem der Ansprüche 1 bis 7, umfassend mindestens 50 Volumenprozent an Trägeröl, vorzugsweise mindestens 80 Volumenprozent, besonders bevorzugt mindestens 95 Volumenprozent, insbesondere mindestens 97 Volumenprozent, wobei der Anteil an Trägeröl maximal 99.89 Volumenprozent beträgt.

9. Zusammensetzung zur Verwendung nach einem der Ansprüche 1 bis 8 zur Behandlung von emotionalen Beschwerden, insbesondere von Traumata, Schock, Stressfolgeerkrankungen, psychischen und somatischen Beschwerden, Angespanntheit, Angst, Antriebslosigkeit, Einsamkeit, emotionalem Schmerz, Erschöpfung, Nervosität, Ohnmacht, Trauer, Überforderung, Unruhe, Verzweiflung und/oder Wut.

## Claims

1. Composition for use in aromatherapy, comprising 0.01 to 0.125 vol.% of an active composition and 0.1 to 99.99 vol.% of a transport composition and optionally a carrier oil and/or an alcohol, wherein
the active composition contains a mixture of 90 to 110 different essential oils and an alcohol in a volume ratio of 1:500 to 1:5000 of oils:alcohol,
the transport composition consists of one or more essential oils,
the carrier oil is selected from the group consisting of avocado oil, calophyllum oil, hemp oil, St. John's wort oil, jojoba oil, coconut fat, macadamia nut oil, almond oil, evening primrose oil, olive oil, calendula oil, sallow thorn oil, black cumin oil, sesame oil, shea butter, grape seed oil, monk's pepper oil, wild rose oil, and mixtures thereof,
the alcohol is selected from the group consisting of methanol, ethanol, propanol, and isopropanol, preferably ethanol,
wherein the essential oils of the active composition are selected from the group consisting of Salvia officinalis, Lavandula stoechas L., Valeriana officinalis, Iris pallida var. florentina, Commiphora myrrha, Commiphora molmol, Osmanthus fragrans, Cedrus atlantica, Zingiber officinalis, Matricaria camomilla, Chamomilla recutita, Leptospermum scoparium, Melissa officinalis CT Citral, Nardostachys jatamansi, Pogostemon cablin, Achillea millefolium L., Vetiveria zizanoides, Juniperus virginiana L., Cananga odorata var. genuina, Daucus carotta L., Santalum album, Santalum austrocaledonicum, Ocimum basilicum, Mentha citrata L., Pelargonium graveolens, Coriandrum sativum L., Bursera delpechiana, Citrus aurantium var. amara, Cymbopogon martinii, Mentha x piperita, Rosa damascena, Aniba parviflora, Aniba rosaeodora, Lavandula spica, Lavandula latifolia, Thymus vulgaris, Thymus officinalis, Thymus vulgaris CT Thujanol-4, Melaleuca cajeputi, Melaleuca leucadendron, Eucalyptus globulus, Eucalyptus radiata, Elettaria cardamomum L., Laurus nobilis L., Melaleuca viridiflora, Melaleuca quinquinervia, Cinnamomum camphora, Rosmarinus officinalis, Thymus mastichina, Hyssopus officinalis var. decumbens, Lippia citriodora Kuntze, Cymbopogon flexuosus, Litsea cubeba, Eucalyptus citriodoria, Angelica archangelica, Citrus aurantium var. bergamia, Cistus ladaniferus L., Pseudotsuga menziesii, Canarium luzonicum A., Artemisia dracunculus L., Abies sibirica, Ferula galbaniflua, Citrus paradisi, Pinus sylvestris, Cuminum cyminum L., Pinus mugo, Citrus aurantifolia, Origanum majorana, Majorana hortensis, Citrus madurensis, Citrus reticulata, Myrtus communis, Myrtus communis CT Myrtenylacetat, Citrus sinensis, Piper nigrum, Abies grandis L., Rosmarinus officinalis, Melaleuca alternifolia, Juniperus communis, Boswellia Carterii, Citrus medica, Citrus limonum, Cupressus sempervirens, Thymus vulgaris, Thymus officinalis, Cinnamomum cassia, Cinnamonum aromaticum, Syzygium aromaticum, Eugenia caryophyllus, Ocimum sanctum L., Cinnamomum ceylanicum, Pimpinella anisum, Foeniculum vulgare var. dulce, Dipteryx odorata, Helichrysum italicum G., Anthemis nobilis, Chamaemelum nobile, Lavandula hybrida, Lavandula officinalis, Lavandula vera, Lavandula angustifolia, Salvia sclarea, Citrus aurantium L. var. pumilia, Boswellia carterii, Betula lenta, Vanilla planifolia, Styrax tonkinensis, Styrax benzoin, Michelia champaca L., Spartium junceum L., Jasminum sambac, Citrus reticulata blanco, Gaultheria fragrantissima, and Cananga odorata var. genuine, and
wherein the essential oils of the transport composition are selected from the group consisting of Vetiveria zizanoides, Daucus carotta L., Nardostachys jatamansi, Santalum album, Santalum austrocaledonicum, Cananga odorata var. genuina, Elettaria cardamomum L., Citrus paradisi, Cymbopogon flexuosus, Litsea Cubeba, Pelargonium graveolens, Melissa officinalis CT Citral, Rosa damascena, Commiphora myrrha, Commiphora molmol, Salvia officinalis, Melaleuca cajeputi, Melaleuca leucadendron, Hyssopus officinalis var. decumbens, Canarium luzonicum A., Ferula galbaniflua, Boswellia Carterii, Cedrus atlantica, and Citrus limonum.

2. Composition for use according to claim 1, wherein the active composition contains essential oils and alcohol in a volume ratio of 1:1000 to 1:2000, preferably in a volume ratio of 1:1300 to 1:1400, and more preferably in a volume ratio of 1:1370 to 1:1380, in particular in a volume ratio of 1:1378.

3. Composition for use according to claim 1 or 2, wherein the active composition comprises a mixture of 90 to 110, preferably of 95 to 100 and more preferably of 98 different essential oils.

4. Composition for use according to one of claims 1 to 3, comprising 0.04 to 0.07 vol.% of the active composition, preferably 0.06 vol.%.

5. Composition for use according to one of claims 1 to 4, wherein the transport composition consists of a mixture of 7 to 35 different essential oils, preferably of 14 to 28 different essential oils, and in particular of 21 different essential oils, wherein the essential oils are contained in equal volume shares.

6. Composition for use according to one of claims 1 to 5, comprising 0.5 to 20 vol.% of the transport composition, preferably 1 to 5 vol.%, more preferably 2.5 to 3 vol.%, and in particular 2.94 vol.%.

7. Composition for use according to one of claims 1 to 6, wherein the active composition and the transport composition contain exclusively natural essential oils.

8. Composition for use according to one of claims 1 to 7, comprising at least 50 vol.% of the carrier oil, preferably at least 80 vol.%, more preferably at least 95 vol.%, in particular at least 97 vol.%, wherein the share of the carrier oil is not more than 99.89 vol.%.

9. Composition for use according to one of claims 1 to 8 for the treatment of emotional disorders, in particular of traumata, shock, sickness caused by stress, psychic and somatic disorders, tension, anxiety, listlessness, loneliness, emotional pain, exhaustion, nervousness, powerlessness, sadness, excessive demands, restlessness, desperation, and/or anger.

## Revendications

1. Composition pour utilisation en aromathérapie, comprenant 0.01 à 0.125 %vol d'une composition active et 0.1 à 99.99 %vol d'une composition de transport et optionnellement une huile de support et/ou un alcool,
la composition active contenant un mélange de 90 à 110 huiles essentielles différentes et un alcool dans un rapport de volume huiles:alcool de 1:500 à 1:5000,
la composition de transport consistant en une ou plusieurs huiles essentielles,
l'huile de support étant sélectionnée dans le groupe consistant en huile d'avocat, huile de calophyllum, huile de chanvre, huile de millepertuis, huile de jojoba, graisse de noix de coco, huile de noix de macadamia, huile d'amande, huile d'onagre, huile d'olive, huile de calendule, huile d'argousier, huile de cumin noir, huile de sésame, beurre de karité, huile de pépins de raisin, huile de vitex, huile de rose sauvage, et des mélanges de celles-ci,
l'alcool étant sélectionné dans le groupe consistant en méthanol, éthanol, propanol, et isopropanol, préférablement éthanol,
les huiles essentielles de la composition active étant sélectionnées dans le groupe consistant en Salvia officinalis, Lavandula stoechas L., Valeriana officinalis, Iris pallida var. florentina, Commiphora myrrha, Commiphora molmol, Osmanthus fragrans, Cedrus atlantica, Zingiber officinalis, Matricaria camomilla, Chamomilla recutita, Leptospermum scoparium, Melissa officinalis CT Citral, Nardostachys jatamansi, Pogostemon cablin, Achillea millefolium L., Vetiveria zizanoides, Juniperus virginiana L., Cananga odorata var. genuina, Daucus carotta L., Santalum album, Santalum austrocaledonicum, Ocimum basilicum, Mentha citrata L., Pelargonium graveolens, Coriandrum sativum L., Bursera delpechiana, Citrus aurantium var. amara, Cymbopogon martinii, Mentha x piperita, Rosa damascena, Aniba parviflora, Aniba rosaeodora, Lavandula spica, Lavandula latifolia, Thymus vulgaris, Thymus officinalis, Thymus vulgaris CT Thujanol-4, Melaleuca cajeputi, Melaleuca leucadendron, Eucalyptus globulus, Eucalyptus radiata, Elettaria cardamomum L., Laurus nobilis L., Melaleuca viridiflora, Melaleuca quinquinervia, Cinnamomum camphora, Rosmarinus officinalis, Thymus mastichina, Hyssopus officinalis var. decumbens, Lippia citriodora Kuntze, Cymbopogon flexuosus, Litsea cubeba, Eucalyptus citriodoria, Angelica archangelica, Citrus aurantium var. bergamia, Cistus ladaniferus L., Pseudotsuga menziesii, Canarium luzonicum A., Artemisia dracunculus L., Abies sibirica, Ferula galbaniflua, Citrus paradisi, Pinus sylvestris, Cuminum cyminum L., Pinus mugo, Citrus aurantifolia, Origanum majorana, Majorana hortensis, Citrus madurensis, Citrus reticulata, Myrtus communis, Myrtus communis CT Myrtenylacetat, Citrus sinensis, Piper nigrum, Abies grandis L., Rosmarinus officinalis, Melaleuca alternifolia, Juniperus communis, Boswellia Carterii, Citrus medica, Citrus limonum, Cupressus sempervirens, Thymus vulgaris, Thymus officinalis, Cinnamomum cassia, Cinnamonum aromaticum, Syzygium aromaticum, Eugenia caryophyllus, Ocimum sanctum L., Cinnamomum ceylanicum, Pimpinella anisum, Foeniculum vulgare var. dulce, Dipteryx odorata, Helichrysum italicum G., Anthemis nobilis, Chamaemelum nobile, Lavandula hybrida, Lavandula officinalis, Lavandula vera, Lavandula angustifolia, Salvia sclarea, Citrus aurantium L. var. pumilia, Boswellia carterii, Betula lenta, Vanilla planifolia, Styrax tonkinensis, Styrax benzoin, Michelia champaca L., Spartium junceum L., Jasminum sambac, Citrus reticulata blanco, Gaultheria fragrantissima, et Cananga odorata var. genuine, et
les huiles essentielles de la composition de transport étant sélectionnées dans le groupe consistant en Vetiveria zizanoides, Daucus carotta L., Nardostachys jatamansi, Santalum album, Santalum austrocaledonicum, Cananga odorata var. genuina, Elettaria cardamomum L., Citrus paradisi, Cymbopogon flexuosus, Litsea Cubeba, Pelargonium graveolens, Melissa officinalis CT Citral, Rosa damascena, Commiphora myrrha, Commiphora molmol, Salvia officinalis, Melaleuca cajeputi, Melaleuca leucadendron, Hyssopus officinalis var. decumbens, Canarium luzonicum A., Ferula galbaniflua, Boswellia Carterii, Cedrus atlantica, et Citrus limonum.

2. Composition pour utilisation selon la revendication 1, la composition active contenant des huiles essentielles et de l'alcool dans un rapport de volume 1:1000 à 1:2000, préférablement dans un rapport de volume 1:1300 à 1:1400, et de façon plus préférée dans un rapport de volume 1:1370 à 1:1380, en particulier dans un rapport de volume 1:1378.

3. Composition pour utilisation selon la revendication 1 ou 2, la composition active comprenant un mélange de 90 à 110, préférablement de 95 à 100 et de façon plus préférée de 98 huiles essentielles différentes.

4. Composition pour utilisation selon l'une des revendications 1 à 3, comprenant 0.04 à 0.07 %vol de composition active, préférablement 0.06 %vol.

5. Composition pour utilisation selon l'une des revendications 1 à 4, la composition de transport consistant en un mélange de 7 à 35 huiles essentielles différentes, préférablement de 14 à 28 huiles essentielles différentes, et en particulier de 21 huiles essentielles différentes, les huiles essentielles étant présentes avec une fraction volumique égale.

6. Composition pour utilisation selon l'une des revendications 1 à 5, comprenant 0.5 à 20 %vol de composition de transport, préférablement 1 à 5 %vol, de façon plus préférée 2.5 à 3 %vol, et en particulier 2.94 %vol.

7. Composition pour utilisation selon l'une des revendications 1 à 6, la composition active et la composition de transport contenant exclusivement des huiles essentielles naturelles.

8. Composition pour utilisation selon l'une des revendications 1 à 7, comprenant au moins 50 %vol d'huile de support, préférablement au moins 80 %vol, de façon plus préférée au moins 95 %vol, en particulier au moins 97 %vol, la fraction d'huile de support étant au maximum 99.89 %vol.

9. Composition pour utilisation selon l'une des revendications 1 à 8 pour le traitement de désordres émotionnels, en particulier traumatismes, chocs, maladies causées par le stress, désordres psychiques et somatiques, tension, anxiété, apathie, solitude, douleurs émotionnelles, épuisement, nervosité, évanouissement, tristesse, surmenage, agitation, désespoir et/ou colère.
